# EUROPEAN PATENT APPLICATION

(11) **EP 4 640 675 A1**
(43) Date of publication of application: **29.10.2025**
(21) Application number: 23906328.2
(22) Date of filing: 05.07.2023
(51) Int. Cl.: C07D 271/10, C01C 3/00

(54) **METHOD FOR PRODUCING 2-AMINO-5-SUBSTITUTED-1,3,4-OXADIAZOLE**

(30) Priority: 22.12.2022 JP 2022205404
(71) Applicant: Mitsubishi Gas Chemical Next Company, Inc., Minato-ku Tokyo 105-7116 (JP)
(72) Inventor: KURODA, Toshihiro, Niigata-shi, Niigata 950-3126 (JP); KAMIMURA, Sadao, Niigata-shi, Niigata 950-3126 (JP)
(74) Representative: Gille Hrabal Partnerschaftsgesellschaft mbB Patentanwälte
(86) International application number: PCT/JP2023/025008
(87) International publication number: WO 2024/134942

(57) **Abstract**

The present invention provides a method for producing a 2-amino-5-substituted-1,3,4-oxadiazole, the method including the following steps (A) to (E). (A) A step of preparing a cyanogen chloride solution from hydrogen cyanide and chlorine. (B) A step of adjusting an amount of byproduct hydrochloric acid secondary produced together with cyanogen chloride in the step (A) to 0.5 equivalents or less relative to the cyanogen chloride produced. (C) A step of subjecting a carboxylic acid hydrazide and the cyanogen chloride solution to a reaction to produce a 2-amino-5-substituted-1,3,4-oxadiazole hydrochloride. (D) A step of adding, to a solution obtained in the step (C), a base in an amount that is equal to or more than an equivalent obtained by adding up an amount of cyanogen chloride and an amount of the byproduct hydrochloric acid in the cyanogen chloride solution used in the step (C). (E) A step of cooling a solution containing a hydrochloride of the base obtained in the step (D) and separating a precipitated crystal.

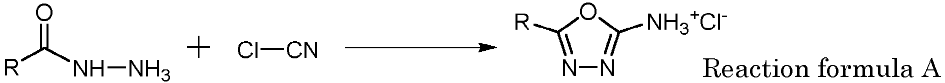

## Description

### Technical Field

The present invention relates to a method for producing an oxadiazole, and, in particular, to a method for producing a 2-amino-5-substituted-1,3,4-oxadiazole.

### Background Art

2-Amino-5-substituted-1,3,4-oxadiazoles are used as an important intermediate for producing pharmaceuticals and agricultural and horticultural herbicides. For example, use thereof is disclosed, in pharmaceuticals, in a metabotropic glutamate receptor antagonist, for treatment or prevention of an acute and chronic disorder of the nervous system, or in an antimicrobial drug for a bacterial disease, and in agrichemicals, as an intermediate of multiple herbicides. Thus, a method for producing a 1,3,4-oxadiazole, which is considered as a useful compound, attract attention. The following synthetic methods have heretofore been known as main methods for producing a 2-amino-5-substituted-1,3,4-oxadiazole.
1) A production method in which semicarbazide and a carboxylic anhydride are reacted to acylate the semicarbazide, followed by simultaneous chlorination and cyclization with phosphoryl chloride, thereby producing a 2-amino-5-substituted-1,3,4-oxadiazole (PTLs 1 and 2)
2) A production method in which semicarbazide and an aldehyde are reacted to convert the semicarbazide to a hydrazone, followed by an electrolytic organic reaction, thereby producing a 2-amino-5-substituted-1,3,4-oxadiazole (NPL 1)
3) A production method in which a carboxylic acid hydrazide is reacted with purified cyanogen chloride or cyanogen bromide to produce a 2-amino-5-substituted-1,3,4-oxadiazole (PTLs 3 and 4, NPL 2)
4) A production method in which a carboxylic acid hydrazide is reacted with phenyl cyanate to produce a 2-amino-5-substituted-1,3,4-oxadiazole (NPL 3)
5) A production method in which a carboxylic acid hydrazide is reacted with trimethylsilyl isothiocyanate to produce a 2-amino-5-substituted-1,3,4-oxadiazole (NPL 4)

### Citation List

### Patent Literature

PTL 1:JP 2017-25054 A
PTL 2:JP 2018-76298 A
PTL 3:EP3133063
PTL 4:JP 2002-542229 T

### Non Patent Literature

NPL 1:Russian Journal of Electrochemistry (2010), 46(1), 34-40
NPL 2:Khimiya Geterotsiklicheskikh Soedinenii (1975), (11), 1493-8
NPL 3:Buletinul Stiintific si Tehnic al Institutului Politehnic Traian Vuia Timisoara, Seria Chimie (1981), 26(2), 111-16
NPL 4:RSC Advances (2013), 3(21), 7684-7687

### Summary of Invention

### Technical Problem

The production method of the above 1) uses semicarbazide which is expensive, and in the step of chlorination and cyclization of the intermediate, phosphoryl chloride is used in an amount that is equal to or more than the equivalent, resulting in a large amount of phosphorus waste liquid and a low yield.

The production method 2) also uses semicarbazide which is expensive as a starting material, and the step of cyclization of the hydrazone obtained through a reaction between semicarbazide and an aldehyde is achieved by an electrocyclic reaction using lithium perchlorate which is expensive. The production is carried out at a low reaction concentration of 1%.

The production method 3) can produce a 2-amino-5-substituted-1,3,4-oxadiazole, but purified cyanogen chloride is to be used and a purification step such as distillation is needed. In addition, since a 2-amino-5-substituted-1,3,4-oxadiazole has a high solubility in water, a complicated operation is needed, for example, in which a carboxylic acid hydrazide and cyanogen chloride are reacted using an organic solvent, then a hydrochloride produced through neutralization is separated by filtration, the filtrate is concentrated and dried into a solid state, and then a small amount of water is added to the residue to crystalize the product. Furthermore, dedicated equipment is needed for the concentration drying. Here, a 2-amino-5-substituted-1,3,4-oxadiazole can be obtained also by using cyanogen bromide, but cyanogen bromide is expensive and a consideration on safety is required because a highly toxic substance is to be handled in the form of crystal.

**In** the production method 4), phenyl cyanate is expensive and the yield of the target product is low.

**In** the production method 5), trimethylsilyl isothiocyanate is expensive and furthermore, hydrogen sulfide which is highly toxic and has an offensive odor is generated as a byproduct of the reaction.

As described above, the conventionally known methods for synthesizing a 2-amino-5-substituted-1,3,4-oxadiazole are not satisfactory as an economic and efficient production method.

The present invention is to solve such a problem involved in the conventional art as described above, and provides an industrial method for producing a 2-amino-5-substituted-1,3,4-oxadiazole, in a high yield and high purity, from a starting material that is inexpensive and is easy to prepare, in a simpler manner as compared with conventional methods.

### Solution to Problem

The present inventors have found that, in a reaction of a cyanogen chloride solution synthesized from hydrogen cyanide and chlorine with a carboxylic acid hydrazide, even without purification of cyanogen chloride, by controlling, in the synthesis of cyanogen chloride from hydrogen cyanide and chlorine, the amount of hydrochloric acid produced as a byproduct together with cyanogen chloride (hereinafter also referred to as "byproduct hydrochloric acid") to 0.5 equivalents or less relative to cyanogen chloride used in the reaction, a 2-amino-5-substituted-1,3,4-oxadiazole hydrochloride can be synthesized without any complicated operation, and that, by adding a base to an aqueous solution of the obtained 2-amino-5-substituted-1,3,4-oxadiazole hydrochloride to precipitate the target product by the salting-out effect of a hydrochloride derived from the byproduct hydrochloric acid produced by addition of the base, followed by solid-liquid separation, a crystal of a 2-amino-5-substituted-1,3,4-oxadiazole can be obtained in a simple manner and in a high purity without removing the hydrochloride produced , thus completing the present invention.

Specifically, the present invention relates to a method for producing a 2-amino-5-substituted-1,3,4-oxadiazole, the method including
(A) a step of preparing a cyanogen chloride solution from hydrogen cyanide and chlorine,
(B) a step of adjusting an amount of byproduct hydrochloric acid secondary produced together with cyanogen chloride in the step (A) to 0.5 equivalents or less relative to the cyanogen chloride produced,
(C) a step of subjecting a carboxylic acid hydrazide represented by the general formula (1) and the cyanogen chloride solution to a reaction to produce a 2-amino-5-substituted-1,3,4-oxadiazole hydrochloride represented by the general formula (2): 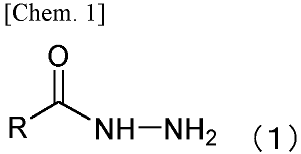 (in the formula, R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group), 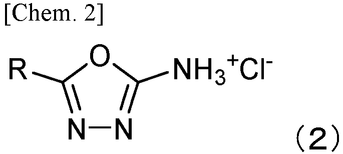 (in the formula, R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group),
(D) a step of adding, to a solution obtained in the step (C), a base in an amount that is equal to or more than an equivalent obtained by adding up an amount of cyanogen chloride and an amount of the byproduct hydrochloric acid in the cyanogen chloride solution used in the step (C),
(E) a step of cooling a solution containing a hydrochloride of the base obtained in the step (D) and separating a precipitated crystal to thereby obtain a 2-amino-5-substituted-1,3,4-oxadiazole represented by the general (3): 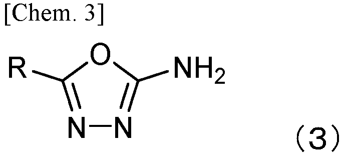
(in the formula, R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group).

**In** other words, the present invention relates to a method for producing a 2-amino-5-substituted-1,3,4-oxadiazole in which the amount of the byproduct hydrochloric acid due to the step (A) in the solution containing the 2-amino-5-substituted-1,3,4-oxadiazole hydrochloride obtained in the step (C) is made to be 0.5 equivalents or less relative to the cyanogen chloride reacted.

### Advantageous Effects of Invention

**In** the method for producing a 2-amino-5-substituted-1,3,4-oxadiazole according to the present invention, it is possible to obtain a 2-amino-5-substituted-1,3,4-oxadiazole in a high yield and high purity by using a cyanogen chloride solution and a carboxylic acid hydrazide which are inexpensive and are easy to prepare and precipitating the target product from a solvent with the produced hydrochloride dissolved therein without a need of a complicated operation, such as separation of the produced hydrochloride or concentration drying. Thus, the present invention provides a simple industrial production method.

### Description of Embodiments

The method for producing a 2-amino-5-substituted-1,3,4-oxadiazole according to the present invention includes the following steps (A) to (E).
(A) A step of preparing a cyanogen chloride solution from hydrogen cyanide and chlorine.
(B) A step of adjusting an amount of byproduct hydrochloric acid secondary produced together with cyanogen chloride in the step (A) to 0.5 equivalents or less relative to the cyanogen chloride produced.
(C) A step of reacting a carboxylic acid hydrazide with cyanogen chloride as shown in the following reaction formula A: 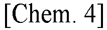 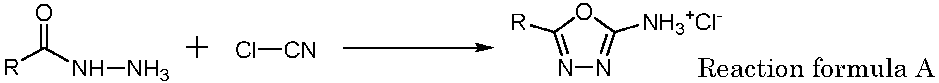 to produce a 2-amino-5-substituted-1,3,4-oxadiazole hydrochloride.
(D) A step of adding, to a solution obtained in the step (C), a base in an amount that is equal to or more than an equivalent obtained by adding up an amount of cyanogen chloride and an amount of the byproduct hydrochloric acid in a cyanogen chloride solution used in the step (C).
(E) A step of cooling a solution containing a hydrochloride of the base obtained in the step (D) and separating a precipitated crystal to thereby obtain a 2-amino-5-substituted-1,3,4-oxadiazole.

Hereinunder, the method for producing a 2-amino-5-substituted-1,3,4-oxadiazole according to the present invention will be specifically described.

### Step (A)

The step (A) is a step of preparing a cyanogen chloride solution from hydrogen cyanide and chlorine.

Cyanogen chloride can be synthesized by reacting hydrogen cyanide and chlorine. **In** this reaction for synthesizing cyanogen chloride from hydrogen cyanide and chlorine, preferably, by reacting hydrogen cyanide of the same equivalent or more with respect to chlorine, byproduct hydrochloric acid is generated in an amount of the same equivalent as the equivalent of the produced cyanogen chloride.

Hydrogen cyanide may be used in the form of gas and liquid of the hydrogen cyanide alone, and hydrogen cyanide previously obtained by a reaction of a metal cyanide and an acid may also be used. As the metal cyanide, for example, potassium cyanide, sodium cyanide, or the like can be used. The metal cyanide can be used in a liquid form (not necessarily have to be dissolved) of a concentration of 5 to 20% by mass obtained by adding the metal cyanide to a solvent, and for example, the liquid is dropwise added to an acidic solution, such as hydrochloric acid, at -5 to 15⁰C, whereby a liquid containing hydrogen cyanide can be obtained. The obtained hydrogen cyanide can be used for a reaction with chlorine in the state as it is mixed in the solvent. The solvent is not particularly limited, and water, an aliphatic hydrocarbon, such as pentane, hexane, or cyclohexane, a halogenated hydrocarbon, such as methylene chloride or chloroform, or the like can be used, and water which has a high solubility of a metal cyanide is preferred. One of the solvents can be used alone or two or more thereof can be used in mixture, and the mixing ratio can be any value.

In the present invention, chlorine used may be in the form of gas or liquid, and it is preferred to blow gaseous chlorine into a reaction liquid.

The amount of hydrogen cyanide is 1 equivalent or more relative to chlorine, and preferably 1.0 to 1.1 equivalents. When an excess amount of chlorine is present in the cyanogen chloride solution, in neutralizing the byproduct hydrochloric acid secondary produced, cyanogen chloride volatilizes while chlorine volatilizes with heat of neutralization, resulting in a loss of cyanogen chloride.

The reaction temperature in the reaction of hydrogen cyanide and chlorine is not particularly limited, but from the viewpoint of preventing the loss of cyanogen chloride due to volatilization, is preferably -10⁰C to 5⁰C.

The reaction pressure in the reaction of hydrogen cyanide and chlorine may be a normal pressure or an increased pressure. The reaction atmosphere is not particularly limited, and may be an atmosphere of an inert gas, such as nitrogen, helium, or argon, according to the need.

The reaction solvent in blowing chlorine is not particularly limited, and an aqueous solution or an aliphatic hydrocarbon, such as pentane, hexane, cyclohexane, heptane, or octane, or a halogenated hydrocarbon, such as methylene chloride or chloroform, can be used. One of the solvents can be used alone or two or more thereof can be used in mixture, and the mixing ratio can be any value.

The time for blowing chlorine into the solution containing hydrogen cyanide is not particularly limited, and is preferably 10 minutes to 20 hours, and more preferably 30 minutes to 15 hours. It is preferred to continue stirring thereafter while keeping the reaction temperature for additional 10 minutes to 1 hour.

### Step (B)

The step (B) is a step of adjusting the amount of the byproduct hydrochloric acid secondary produced together with cyanogen chloride in the step (A) to 0.5 equivalents or less relative to the cyanogen chloride produced. Here, to adjust the amount to 0.5 equivalents or less relative to the cyanogen chloride produced means that, with the equivalent of the produced cyanogen chloride, i.e., the equivalent of the byproduct hydrochloric acid secondary produced in an amount of the same equivalent as the equivalent of the produced cyanogen chloride taken as 1.0, the amount of the byproduct hydrochloric acid is made to be 0.5 equivalents or less relative to the former equivalent, and thus, means that the equivalent of the byproduct hydrochloric acid is made to be an equivalent that is half or less of the equivalent of the produced cyanogen chloride.

**In** the present invention, the amount of the byproduct hydrochloric acid in the cyanogen chloride solution in reacting a carboxylic acid hydrazide and the cyanogen chloride solution is important. The amount is preferably 0.5 equivalents or less relative to the produced cyanogen chloride, and further preferably 0.4 equivalents or less. When the byproduct hydrochloric acid is contained in an amount of 0.5 equivalents or more, the reaction rate decreases, resulting in significantly reduced yield of the 2-amino-5-substituted-1,3,4-oxadiazole hydrochloride. **In** contrast, in the case of 0.5 equivalents or less, a sufficient reaction rate is given, and it is not necessary to use cyanogen chloride obtained through purification such as distillation.

**In** addition, the whole amount of the byproduct hydrochloric acid may be removed, but when the byproduct hydrochloric acid is removed by neutralization, neutralization with a base of an amount that is equal to or more than the amount of the byproduct hydrochloric acid leads to decomposition of the produced cyanogen chloride by the base, and thus, neutralization with a base in an amount that is equal to or more than the amount of the byproduct hydrochloric acid is not preferred.

For making the amount of the byproduct hydrochloric acid in the cyanogen chloride solution used in the step (C) which is the next step be 0.5 equivalents or less relative to the produced cyanogen chloride, the following methods are exemplified.

A method in which the cyanogen chloride solution obtained in the step (A) is subjected to a simple treatment, for example, neutralization or extraction, to make the amount of byproduct hydrochloric acid be 0.5 equivalents or less relative to the cyanogen chloride, and the resultant is used; and a method in which an untreated cyanogen chloride solution (that is, one containing the byproduct hydrochloric acid in an amount of 0.5 equivalents or more) is used and the cyanogen chloride solution, a carboxylic acid hydrazide, and a base are mixed at once or the cyanogen chloride solution and a carboxylic acid hydrazide are mixed, and then, a base is mixed therewith, followed by reacting the cyanogen chloride solution and the carboxylic acid hydrazide.

Specific examples of methods for making the amount of the byproduct hydrochloric acid in the cyanogen chloride solution obtained in the step (A) be 0.5 equivalents or less relative to cyanogen chloride include a method in which a base is added in the absence or presence of an organic solvent to convert a part of the byproduct hydrochloric acid to the hydrochloride thereof (hereinafter also referred to as "neutralization method") and a method in which an organic solvent is added to extract cyanogen chloride into the organic solvent, whereby making the amount of the byproduct hydrochloric acid be 0.5 equivalents or less (hereinafter also referred to as "extraction method").

In the case of a neutralization method, by adding a base to the cyanogen chloride solution prepared from hydrogen cyanide and chlorine, or by adding a base simultaneously with or after mixing a carboxylic acid hydrazide and the cyanogen chloride solution, to neutralize a part of the byproduct hydrochloric acid, a salt of the byproduct hydrochloric acid can be formed. In general, the salt generated in neutralization is removed from the reaction system, but in the present invention, it is preferred that the salt is not removed but the resultant is used as it is in the state containing the byproduct hydrochloric acid salt.

The base used in neutralization of the byproduct hydrochloric acid secondary produced in the synthesis of cyanogen chloride is not particularly limited, and examples thereof include alkali metal hydroxides, such as sodium hydroxide and potassium hydroxide, an alkaline earth metal hydroxide, such as calcium hydroxide, alkali metal carbonates, such as sodium carbonate and potassium carbonate, and organic bases, such as triethylamine and pyridine. The carboxylic acid hydrazide which is a raw material may also be used as a base. The form of the base used is not limited, and the base may be used as an aqueous solution in which the base is dissolved in water. One of the bases may be used alone or two or more thereof may be used in combination.

The equivalent of the base used for neutralization of the byproduct hydrochloric acid is in the range of 0.5 to 1.0 equivalent relative to the byproduct hydrochloric acid (that is, relative to the produced cyanogen chloride), and preferably 0.6 equivalents or more. When the equivalent of the base is 0.5 equivalents or less, the amount of the hydrochloric acid that does not forms a salt after the reaction of the step (C), that is, the byproduct hydrochloric acid is 0.5 equivalents or more relative to the cyanogen chloride used for the reaction, and thus, the reaction is delayed to significantly lower the yield. On the other hand, addition of a base of the equivalent or more causes decomposition of cyanogen chloride, and also in this case, the yield of the 2-amino-5-substituted-1,3,4-oxadiazole hydrochloride decreases.

The solvent in the neutralization of the byproduct hydrochloric acid described above may be only water, but preferably contains an organic solvent. The organic solvent is not particularly limited, and an aliphatic hydrocarbon, such as pentane, hexane, cyclohexane, heptane, octane, or decane, a halogenated hydrocarbon, such as methylene chloride, chloroform, trichloroethylene, or tetrachloroethylene, or the like can be used. The amount of the organic solvent added is 0.6 to 7.0 times by weight relative to the weight of the hydrogen cyanide used. The organic solvent may be newly added in neutralization, but the solvent used in the synthesis of cyanogen chloride in the step (A) may be used, and this may replace the addition of an organic solvent in neutralization. The organic solvent used herein is used for preventing the volatilization loss of cyanogen chloride, and thus, when no organic solvent is used or when no organic solvent is added in neutralization, the yield of the target product may sometimes decrease because of the loss due to the volatilization of cyanogen chloride in neutralization. **In** this point, the organic solvent is different in the action and effect from an organic solvent used for extraction in the "extraction method" described below.

The neutralization temperature of the byproduct hydrochloric acid described above is in the range of **-10** to 50⁰C, and preferably in the range of -5⁰C to 20⁰C.

The time of dropwise addition of the base used for neutralization is in the range of 10 minutes to 20 hours, and preferably in the range of 1 hour to 15 hours.

The cyanogen chloride solution in which the amount of the byproduct hydrochloric acid is decreased by addition of a base in this manner is subjected to a reaction with a carboxylic acid hydrazide, when an organic solvent is present, as it is a solution of a two-layer system containing the organic solvent.

**In** addition, when as the cyanogen chloride solution, one containing 0.5 equivalents or more of the byproduct hydrochloric acid is used, the cyanogen chloride solution, a carboxylic acid hydrazide, and a base are mixed together, or the cyanogen chloride solution is mixed with a carboxylic acid hydrazide, and then a base is mixed therewith, to lower the amount of the byproduct hydrochloric acid to 0.5 equivalents or less, followed by a reaction between a carboxylic acid hydrazide and the cyanogen chloride solution of the next step, thus synthesizing a 2-amino-5-substituted-1,3,4-oxadiazole hydrochloride.

On the other hand, in the case of the extraction method, from a cyanogen chloride preparation liquid containing water (in which water is added after synthesis under an organic solvent), cyanogen chloride is extracted with an organic solvent, followed by liquid separation, whereby the amount of the byproduct hydrochloric acid secondary produced can be decreased.

The organic solvent used for extracting cyanogen chloride from the cyanogen chloride solution synthesized from hydrogen cyanide and chlorine is preferably a halogenated hydrocarbon, such as methylene chloride or tetrachloroethylene. One of the bases may be used alone or two or more thereof may be used in combination. The extraction solvent may be contained in the system in the synthesis of cyanogen chloride. The amount of the organic solvent added is not particularly limited, but preferably 1.0 to 8.0 times by weight relative to the weight of hydrogen cyanide used, and preferably 3.0 to 6.0 times by weight.

The extraction temperature is not limited, and in general, is -5 to 10⁰C. Subsequently, using the organic solvent used for extraction and liquid separation, a reaction with a carboxylic acid hydrazide is carried out.

In the present invention, preparing the amount of the byproduct hydrochloric acid can be achieved by using the neutralization method or extraction method described above alone. However, the present invention is not limited thereto but both of the methods may be used in combination to prepare the amount of the byproduct hydrochloric acid. For example, when the amount of the byproduct hydrochloric acid in an extraction liquid obtained by an extraction method is more than 0.5 equivalents, the amount of the byproduct hydrochloric acid can be prepared by a neutralization method in which a base is added to the extraction liquid. Alternatively, the amount of the byproduct hydrochloric acid can also be prepared by subjecting the extraction liquid to additional extraction.

### Step (C)

The step (C) is a step of reacting a carboxylic acid hydrazide and cyanogen chloride to synthesize a 2-amino-5-substituted-1,3,4-oxadiazole hydrochloride.

Through a reaction between the carboxylic acid hydrazide and cyanogen chloride, a 2-amino-5-substituted-1,3,4-oxadiazole and hydrochloric acid are produced, and the produced hydrochloric acid forms a salt with the 2-amino-5-substituted-1,3,4-oxadiazole, resulting in production of the 2-amino-5-substituted-1,3,4-oxadiazole hydrochloride. Accordingly, the solution after completion of the reaction contains the 2-amino-5-substituted-1,3,4-oxadiazole hydrochloride and a part of the byproduct hydrochloric acid secondary produced in the step (A).

The carboxylic acid hydrazide which is reacted with cyanogen chloride is a compound represented by the following general formula (1). The carboxylic acid hydrazide may be used as the compound as it is, but from the viewpoint of controlling the temperature in the operation of dropwise addition, is preferably used as an aqueous solution or a solution in other organic solvent. In general, an aqueous solution of approximately 5 to 80% by mass is used in many cases.

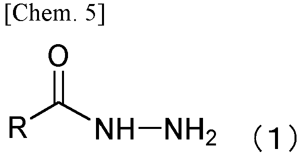

**In** the formula, the substituent R is a hydrogen atom, a C1 to C6 alkyl group, an aryl group, a 5- or 6-membered aromatic heterocyclic group, or an alkoxy group, and these groups may be substituted with halogen or a C1 to C4 alkyl group.

The alkyl group having 1 to 6 carbon atoms, i.e., the C1 to C6 alkyl group includes both a linear alkyl group and a branched alkyl group, and the alkyl group is preferably a methyl group.

Examples of the aryl group include a phenyl group and a naphthyl group, and a phenyl group is preferred.

The 5- or 6-membered aromatic heterocyclic group means a monocyclic aromatic heterocyclic ring in which the number of ring atoms is 5 or 6 and the ring atoms contain 1 to 5 heteroatoms (the heteroatom means a nitrogen atom, an oxygen atom, or a sulfur atom), such as a pyridyl group, a furanyl group, or a thienyl group. The 5- or 6-membered aromatic heterocyclic group is preferably a pyridyl group.

Examples of the alkoxy group include so-called alkoxy groups, such as a methoxy group, an ethoxy group, and an isopropoxy group, and also include a phenoxy group and the like. The alkoxy group is preferably a methoxy group.

The groups mentioned above may be substituted with halogen or a C1 to C4 alkyl group, but since an increase in the number of carbon atoms of R in the formula leads to a decrease in the reactivity, the upper limit of the number of carbon atoms is preferably C18. Examples of the substituent include a tolyl group in which a methyl group is substituted on a phenyl group and a haloalkyl group in which halogen is substituent on an alkyl group (such as fluorine, chlorine, and bromine).

The carboxylic acid hydrazide can be easily synthesized by reacting hydrazine with a carboxylic acid ester, a carboxylic acid chloride, a carboxylic acid anhydride, a carboxylic acid ester, a carbamic acid ester. The form of the carboxylic acid hydrazide used is not limited, and a synthetic liquid of the carboxylic acid hydrazide, a concentrated liquid obtained by a concentration operation, or a crystal of the carboxylic acid hydrazide may be used. The carboxylic acid hydrazide may be distilled with a solvent as needed, and the dilution solvent is not particularly limited, and examples thereof include water, alcohols, such as methanol, ethanol, and isopropanol, aliphatic hydrocarbons, such as pentane, hexane, cyclohexane, heptane, and octane, aromatic hydrocarbons, such as toluene and xylene, ethers, such as diethyl ether and diisopropyl ether, and halogenated hydrocarbons, such as methylene chloride and chloroform.

A product obtained by reacting cyanogen chloride and the carboxylic acid hydrazide of the above formula (1) is a 2-amino-5-substituted-1,3,4-oxadiazole hydrochloride represented by the general formula (2).

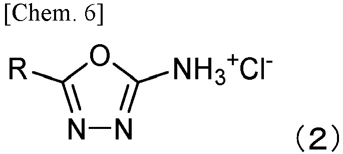

In the formula, the substituent R is derived from the R in the carboxylic acid hydrazide and is the same as the R. Specifically, R is a hydrogen atom, a C1 to C6 alkyl group, an aryl group, a 5- or 6-membered aromatic heterocyclic group, and an alkoxy group, and these groups may be substituted with halogen or a C1 to C4 alkyl group.

In the present invention, the reaction solvent in the reaction between cyanogen chloride and the carboxylic acid hydrazide is not particularly limited, and, for example, water, an aliphatic hydrocarbon, such as pentane, hexane, cyclohexane, heptane, or octane, or a halogenated hydrocarbon, such as methylene chloride or chloroform, can be used. One of the solvents can be used alone or two or more thereof can be used in mixture, and the mixing ratio can be any value. In view of the economy and environmental impact, water solvent is preferred.

In the present invention, as the amount of cyanogen chloride increases, the amount of a base to be added in the step (D) described later increases. As the amount of cyanogen chloride is more excessive relative to the carboxylic acid hydrazide, the amount of water for dissolving the hydrochloride of the base increases and the productivity decreases, and thus, it is preferred to set the equivalent of the carboxylic acid hydrazide relative to the cyanogen chloride so as to efficiently consume cyanogen chloride.

In the present invention, the equivalent of the carboxylic acid hydrazide is preferably 0.95 to 1.5 equivalents relative to cyanogen chloride. When the equivalent of the carboxylic acid hydrazide is 0.95 or less, the productivity decreases. On the other hand, an equivalent of 1.5 or more is not superior from the viewpoint of the economy.

In the present invention, the charging method in the reaction between the cyanogen chloride solution and the carboxylic acid hydrazide is not limited, but is preferably a dropwise addition method because the reaction is accompanied with heat generation. For the dropwise addition, a carboxylic acid hydrazide solution may be dropwise added to the cyanogen chloride solution having an amount of the byproduct hydrochloric acid reduced to 0.5 equivalents or less, or the cyanogen chloride solution may be dropwise added to a carboxylic acid hydrazide solution.

On the other hand, when a cyanogen chloride solution containing the byproduct hydrochloric acid in an amount of 0.5 equivalents or more relative to cyanogen chloride is used, a method in which the cyanogen chloride solution is dropwise added to a carboxylic acid hydrazide solution and the amount of the byproduct hydrochloric acid is made to be 0.5 equivalents or less at the beginning is adopted, or a carboxylic acid hydrazide solution and a base are dropwise added simultaneously to the cyanogen chloride solution, or a method in which a carboxylic acid hydrazide solution is dropwise added to the cyanogen chloride solution and then, a base is added can also be adopted.

In the present invention, the temperature in dropwise addition of the carboxylic acid hydrazide solution into the cyanogen chloride solution is in the range of -10⁰C to 15⁰C, preferably in the range of -7⁰C to 10⁰C, and more preferably in the range of -5⁰C to 5⁰C. The time for dropwise addition is set so that the temperature can be kept, and is in the range of 10 minutes to 20 hours, and preferably 30 minutes to 15 hours. This is the same also in the case where the cyanogen chloride solution is dropwise added to the carboxylic acid hydrazide solution.

It is preferred that, after completion of dropwise addition, the temperature is increased to 5⁰C to 40⁰C, preferably 10⁰C to 35⁰C, more preferably 20⁰C to 30⁰C, and the solution is stirred while keeping the temperature for 30 minutes to 3 hours, preferably for 1 hour to 2 hours. The time for temperature rising after completion of dropwise addition is not particularly limited, but is in the range of 10 minutes to 15 hours, preferably in the range of 30 minutes to 10 hours, and more preferably in the range of 1 hour to 6 hours.

### Step (D)

The step (D) is a step of adding a base to the reaction liquid obtained in the step (C) to thereby neutralize the byproduct hydrochloric acid contained in the reaction liquid while converting the 2-amino-5-substituted-1,3,4-oxadiazole hydrochloride to a 2-amino-5-substituted-1,3,4-oxadiazole. Accordingly, the amount of the base added is the equivalent corresponding to the sum of the amount of cyanogen chloride in the cyanogen chloride solution used in the reaction in the step (C) (this amount corresponds to the amount of the 2-amino-5-substituted-1,3,4-oxadiazole hydrochloride produced) and the amount of the byproduct hydrochloric acid remaining in the cyanogen chloride solution or more.

In the present invention, in both the methods for reducing the byproduct hydrochloric acid after synthesizing cyanogen chloride (a treatment by a neutralization method and a treatment by an extraction method), the organic solvent contained in the reaction liquid after the reaction of the step (C) is removed by liquid separation so that the organic solvent is not contained in isolation of the product. When the hydrochloride is precipitated in the reaction liquid, water may be added to dissolve the hydrochloride.

When the byproduct hydrochloric acid in preparation of the cyanogen chloride solution in the step (A) is reduced by a neutralization method, the organic solvent used in neutralization is contained in the reaction solution obtained in the step (C), and thus, the organic layer is separated from the reaction liquid obtained in the step (C), and the aqueous layer is treated with a base to neutralize the remaining byproduct hydrochloric acid and convert the 2-amino-5-substituted-1,3,4-oxadiazole hydrochloride to the 2-amino-5-substituted-1,3,4-oxadiazole.

On the other hand, when a cyanogen chloride solution obtained by an extraction method is used, water is added after the reaction with the carboxylic acid hydrazide solution to dissolve a crystal to form a two-layer state of an organic layer and an aqueous layer, and then, the organic layer is separated and the aqueous layer is treated with a base.

When the organic layer is separated and the aqueous layer is treated with a base as described above, the base may be dropwise added to the aqueous layer, or a so-called reverse dropwise addition neutralization may be adopted in which the aqueous layer is dropwise added to the base.

The temperature in the liquid separation of the organic layer and the aqueous layer here is not particularly limited, but is preferably in the range of 5⁰C to 40⁰C, and more preferably in the range of 10⁰C to 30⁰C.

In the present invention, the base used in the step (D) is not particularly limited, and examples thereof include alkali metal hydroxides, such as sodium hydroxide and potassium hydroxide, an alkaline earth metal hydroxide, such as calcium hydroxide, alkali metal carbonates, such as sodium carbonate and potassium carbonate, and organic bases, such as triethylamine and pyridine. The form of the base used is not limited, and the base may be used as an aqueous solution in which the base is dissolved in water. One of the bases may be used alone or two or more thereof may be used in combination. In the treatment with the base, the base may be dropwise added to the reaction liquid, or the reaction liquid can be dropwise added to the base as described above.

In the present invention, the treatment temperature in the treatment with the base is in the range of 5⁰C to 80⁰C, and preferably in the range of 5⁰C to 40⁰C. The time for neutralization is in the range of 10 minutes to 20 hours, and preferably in the range of 20 minutes to 15 hours.

In the present invention, the cyanogen chloride solution prepared in the step (A) contains the byproduct hydrochloric acid. Then, in the step (C), a 2-amino-5-substituted-1,3,4-oxadiazole hydrochloride of the same equivalent as the equivalent of cyanogen chloride is produced. The byproduct hydrochloric acid contained in the cyanogen chloride solution is removed by a neutralization method or an extraction method, or in the case of untreated as described above, by a method in which the carboxylic acid hydrazide and the base are reacted together. Accordingly, the equivalent of the base added in the step (D) is required to be the equivalent obtained by adding up the byproduct hydrochloric acid contained in the cyanogen chloride solution obtained after any of the treatments described above and the 2-amino-5-substituted-1,3,4-oxadiazole hydrochloride produced in the step (C) or more. When the equivalent of the base added is the equivalent of hydrochloric acid obtained by the adding up as described above or less, the 2-amino-5-substituted-1,3,4-oxadiazole remains in the form of hydrochloride, and thus the yield significantly decreases. Also for completely decomposing the unreacted cyanogen chloride to enable safe handling, the base is desirably added in an amount of the added-up equivalent as described above or more. Furthermore, when the base of the same equivalent as the added-up equivalent of hydrochloric acid is added, a colored crystal tends to be obtained. In contrast, when the base is added in an amount of the added-up equivalent or more, a white crystal can be obtained. From the above description, the amount of the base added is more preferably in the range of 1.1 to 3.0 equivalents relative to the total amount of the byproduct hydrochloric acid contained in the cyanogen chloride solution of the step (C) and the cyanogen chloride used in the step (C), and further preferably in the range of 1.2 to 1.5 equivalents. A hydrochloride produced is generally removed from the reaction system, but in the present invention, the produced hydrochloride is not removed in order to obtain the salting-out effect and is used in the step (E) in the dissolved state.

In the present invention, in the step (D) of adding a base, that is, the step of neutralization with a base, the 2-amino-5-substituted-1,3,4-oxadiazole hydrochloride is converted to the 2-amino-5-substituted-1,3,4-oxadiazole while the remaining byproduct hydrochloric acid is neutralized, and thus, the purpose of the neutralization is achieved as long as the pH of the liquid after the neutralization treatment is a value of the pH when the neutralization reaction is completed, that is, a pH of about 4.5 or higher, and in the liquid after the neutralization treatment, the 2-amino-5-substituted-1,3,4-oxadiazole is produced, and the 2-amino-5-substituted-1,3,4-oxadiazole can be obtained by crystallization. However, in the present invention, in light of the sequential steps, a cyanide produced in the treatment (for example, NaCN) gives a pH of a solution of the cyanide itself as high as 11 to 12, and at pH11 or lower, the cyanide may remain as HCN. Thus, for safely treating a cyanide, the pH of the liquid after the neutralization treatment is needed to be 11 or higher. Accordingly, the pH of the liquid after the neutralization treatment is preferably pH11 or higher. Also from this, a base of the equivalent or more is needed as described above.

### Step (E)

The step (E) is a step of precipitating the target product by the salting-out effect of the hydrochloride of the base produced in the step (D) (that is, the base used in neutralization), followed by solid-liquid separation, to thereby obtain a 2-amino-5-substituted-1,3,4-oxadiazole represented by the general formula (3) without removing the hydrochloride produced as a byproduct.

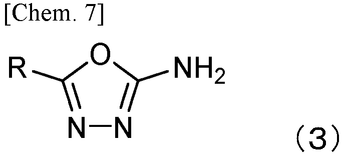

In the formula, the substituent R is derived from the R in the carboxylic acid hydrazide and is the same as the R. Specifically, R is a hydrogen atom, a C1 to C6 alkyl group, an aryl group, a 5- or 6-membered aromatic heterocyclic group, and an alkoxy group, and these groups may be substituted with halogen or a C1 to C4 alkyl group.

In the present invention, although a 2-amino-5-substituted-1,3,4-oxadiazole has a very high solubility in water, in an aqueous solution containing a salt, the solubility decreases by the salting-out effect, making it possible to obtain the product in a high yield. As an example of the salting-out effect, 2-amino-5-methyl-1,3,4-oxadiazole has a solubility in water at 30⁰C of 13.9% by mass, but in a 10 mass% aqueous NaCl solution, the solubility of 2-amino-5-methyl-1,3,4-oxadiazole decreases to 7.9% by mass, and in a 26.4 mass% aqueous NaCl solution, the solubility decreases to 3.5% by mass. Based on the above fact, the concentration of the hydrochloride based on the base added in the solution obtained by the step (D) is preferably 10% by mass or more, and is preferably the saturated concentration of the hydrochloride of the used base or lower.

In the present invention, the solubility of a 2-amino-5-substituted-1,3,4-oxadiazole in an aqueous solution (containing the hydrochloride of the base added) is highly dependent on the temperature, and as a lower separation temperature gives a higher yield. In an aqueous solution containing a hydrochloride of a base, separation can be performed at 0⁰C or lower owing to the depression of freezing point. For example, the solubility of 2-amino-5-methyl-1,3,4-oxadiazole in an aqueous 26.4 mass% NaCl solution at 30⁰C is 3.5% by mass, but the solubility is lowered to 1.1% by mass when the temperature is decreased to -5⁰C. On the other hand, when the separation temperature is too low, impurities are mixed in, and thus, the separation temperature is in the range of -7⁰C to 30⁰C, and preferably in the range of -5⁰C to 10⁰C. The cooling time to the separation temperature is not particularly limited, but is in the range of 10 minutes to 15 hours, and preferably in the range of 30 minutes to 10 hours.

In the present invention, the crystal separated from the solution containing the hydrochloride of the base obtained in the step (D) contains the hydrochloride of the base, and thus, is preferably washed with water or an organic solvent containing water. After washed with water or an organic solvent containing water, the content of the hydrochloride in the crystal is 0.5% by mass or less. In addition, washing with water may be followed by washing with an organic solvent. The temperature of the washing liquid, in the case of water, is in the range of 0⁰C to 20⁰C, and preferably in the range of 0⁰C to 10°C. In the case of an organic solvent containing water, the temperature is in the range of -10°C to 20⁰C, and preferably in the range of -5⁰C to 10°C.

The pH of the liquid containing the salt of the byproduct hydrochloric acid after the neutralization treatment, that is, the liquid for crystallization, is preferably pH11 or higher as described above.

In the present invention, by washing and then, drying the separated crystal, a 2-amino-5-substituted-1,3,4-oxadiazole represented by the general formula (3) can be obtained. As described above, a simple production method using raw materials that are inexpensive and are easy to prepare is provided. Accordingly, this method is such a superior production method that is industrially implemented.

### Examples

The present invention will be described in more detail below based on Examples, but the present invention is not to be limited to the Examples.

### (Example 1)

### ·Acetohydrazide synthetic step

Into a four-neck flask, 120.0 g (2.517 mol) of 100% hydrazine hydrate was put, and 221.8 g of ethyl acetate (1.05 equivalents / hydrazine hydrate) was dropwise added under room temperature. After dropwise addition, the reaction liquid was heated to 77.0⁰C, and was subjected to a reaction under reflux for 7 hours. After completion of the reaction, the reaction liquid was concentrated under a reduced pressure to obtain 215.5 g (yield 93.5%) of an acetohydrazide concentrated liquid. Gas chromatography analysis revealed that the acetohydrazide content in the concentrated liquid was 77.0% by mass. In the next step, the scale was adjusted so that the weight of the concentrated liquid used was 64.9 g.

### ·2-Amino-5-methyl-1,3,4-oxadiazole synthetic step

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of a 48% aqueous sodium hydroxide solution (0.80 equivalents / cyanogen chloride) was dropwise added within -5 to 0⁰C over 1 hour to adjust the byproduct hydrochloric acid in the reaction liquid to 0.20 equivalents / cyanogen chloride. After neutralization of the byproduct hydrochloric acid, 64.9 g of the 77.0% acetohydrazide concentrated liquid (0.96 equivalents / hydrogen cyanide) was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The amount of hydrochloric acid that did not form a salt on the completion of the reaction, that is, the byproduct hydrochloric acid in the step (A) was 0.20 equivalents relative to cyanogen chloride used in the reaction. After completion of the reaction, the reaction liquid was allowed to stand for 15 minutes.

Then, into a four-neck flask, 77.9 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 48.7 g (yield 70.0%) of a white crystal of 2-amino-5-methyl-1,3,4-oxadiazole was obtained. The content of the product was 99.7% by mass, and the content of sodium chloride was 0.2% by mass.

### (Example 2)

In a separable flask, 19.0 g of hydrogen cyanide (0.703 mol) was blown into 130.3 g of water and 28.7 g of methylene chloride and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of 48% sodium hydroxide (0.80 equivalents / cyanogen chloride) was dropwise added within -5 to 0⁰C over 1 hour to adjust the byproduct hydrochloric acid in the reaction liquid to 0.20 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.20 equivalents relative to cyanogen chloride used in the reaction. After completion of the reaction, the reaction liquid was allowed to stand for 15 minutes.

Then, into a four-neck flask, 77.9 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 48.5 g (yield 69.7%) of a white crystal of 2-amino-5-methyl-1,3,4-oxadiazole was obtained. The content of the product was 99.6% by mass, and the content of sodium chloride was 0.3% by mass.

### (Example 3)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 53.3 g of an 48% aqueous sodium hydroxide (0.90 equivalents / cyanogen chloride) was dropwise added within -5 to 0⁰C over 1 hour to adjust the byproduct hydrochloric acid in the reaction liquid to 0.10 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.10 equivalents relative to cyanogen chloride used in the reaction. After completion of the reaction, the reaction liquid was allowed to stand for 15 minutes.

Then, into a four-neck flask, 72.0 g of a 48% aqueous sodium hydroxide solution (1.23 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 47.2 g (yield 67.8%) of a white crystal of 2-amino-5-methyl-1,3,4-oxadiazole was obtained. The content of the product was 99.4% by mass, and the content of sodium chloride was 0.2% by mass.

### (Example 4)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 41.0 g of 48% sodium hydroxide (0.70 equivalents / cyanogen chloride) was dropwise added within -5 to 0⁰C over 1 hour to adjust the byproduct hydrochloric acid in the reaction liquid to 0.30 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.30 equivalents relative to cyanogen chloride used in the reaction. After completion of the reaction, the reaction liquid was allowed to stand for 15 minutes.

Then, into a four-neck flask, 83.4 g of a 48% aqueous sodium hydroxide solution (1.42 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 48.6 g (yield 69.8%) of a white crystal of 2-amino-5-methyl-1,3,4-oxadiazole was obtained. The content of the product was 99.5% by mass, and the content of sodium chloride was 0.2% by mass.

### (Example 5)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 32.2 g of 48% sodium hydroxide (0.55 equivalents / cyanogen chloride) was dropwise added within -5 to 0⁰C over 1 hour to adjust the byproduct hydrochloric acid in the reaction liquid to 0.45 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.45 equivalents relative to cyanogen chloride used in the reaction. After completion of the reaction, the reaction liquid was allowed to stand for 15 minutes.

Then, into a four-neck flask, 92.5 g of a 48% aqueous sodium hydroxide solution (1.58 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 42.5 g (yield 61.0%) of a white crystal of 2-amino-5-methyl-1,3,4-oxadiazole was obtained. The content of the product was 99.2% by mass, and the content of sodium chloride was 0.3% by mass.

### (Example 6)

In a separable flask, 71.2 g of conc. hydrochloric acid (0.703 mol), 117.5 g of water, and 28.7 g of hexane were put, and the resultant was cooled to -5⁰C. After cooling, 125.7 g of a 27.8% aqueous sodium cyanide solution (0.703 mol) was dropwise added within -5.0 to 5⁰C. After dropwise addition, 49.1 g of chlorine (0.985 equivalents / sodium cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of 48% sodium hydroxide (0.80 equivalents / cyanogen chloride) was dropwise added within -5 to 0⁰C over 1 hour to adjust the byproduct hydrochloric acid in the reaction liquid to 0.20 equivalents / cyanogen chloride. After neutralization of the byproduct hydrochloric acid, 64.9 g of a 77.0% acetohydrazide concentrated liquid (0.96 equivalents / sodium cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.20 equivalents relative to cyanogen chloride used in the reaction. After completion of the reaction, the reaction liquid was allowed to stand for 15 minutes.

Then, into a four-neck flask, 77 g of a 48% aqueous sodium hydroxide solution (1.33 equivalents / sodium cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 45.9 g (yield 66.1% / sodium cyanide) of a white crystal of 2-amino-5-methyl-1,3,4-oxadiazole was obtained. The content of the product was 99.5% by mass, and the content of NaCl was 0.3% by mass.

### (Example 7)

### ·Acetohydrazide synthetic step

Into a four-neck flask, 120.0 g (2.517 mol) of 100% hydrazine hydrate was put, and 221.8 g of ethyl acetate (1.05 equivalents / hydrazine hydrate) was dropwise added under room temperature. After dropwise addition, the reaction liquid was heated to 77.0⁰C, and was subjected to a reaction under reflux for 7 hours. After completion of the reaction, the reaction liquid was concentrated under a reduced pressure to obtain 215.5 g (yield 93.5%) of an acetohydrazide concentrated liquid. Gas chromatography analysis revealed that the acetohydrazide content was 77.0% by mass. To the obtained acetohydrazide concentrated liquid, 162.5 g of water was added to adjust the content to 43.9% by mass. In the next step, the scale was adjusted so that the weight of the liquid used was 120.6 g.

### ·2-Amino-5-methyl-1,3,4-oxadiazole synthetic step

In a separable flask, 20.1 g of hydrogen cyanide (0.745 mol) was blown into 83.6 g of water and 123.5 g of trichloroethylene and the resultant was cooled to -5⁰C. After cooling, 51.9 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.718 mol of cyanogen chloride was produced. After completion of the reaction, the organic layer was taken into a four-neck flask. Next, 62.6 g of trichloroethylene was added to the aqueous layer. The mixture was stirred for 15 minutes, and was allowed to stand, and the organic layer was taken and was mixed with the first organic layer. This operation was repeated 5 times. To the separated organic layer, 120.6 g of the 43.9% aqueous acetohydrazide solution (0.96 equivalents / hydrogen cyanide) was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.19 equivalents relative to cyanogen chloride used in the reaction. After completion of the reaction, 50.1 g of water was added, and the mixture was stirred for 15 minutes and was allowed to stand for 15 minutes, and the organic layer was taken and discarded.

Then, into a four-neck flask, 93.1 g of a 48% aqueous sodium hydroxide solution (1.50 equivalents / hydrogen cyanide) was put, and was stirred at room temperature, and the aqueous layer of the reaction liquid was dropwise added to the aqueous sodium hydroxide solution in the four-neck flask within 10 to 40⁰C to perform neutralization. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 51.7 g (yield 70.0%) of a white crystal of 2-amino-5-methyl-1,3,4-oxadiazole was obtained. The content of the product was 99.5% by mass, and the content of sodium chloride was 0.3% by mass.

### (Example 8)

In a separable flask, 20.0 g of hydrogen cyanide (0.739 mol) was blown into 84.5 g of water and 125.5 g of methylene chloride and the resultant was cooled to -5⁰C. After cooling, 51.6 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.728 mol of cyanogen chloride was produced. After completion of the reaction, the organic layer was taken into a four-neck flask. Next, 62.7 g of methylene chloride was added to the aqueous layer. The mixture was stirred for 15 minutes, and was allowed to stand, and the organic layer was taken and was mixed with the first organic layer. This operation was repeated 2 times. To the organic layer separated, 120.6 g of a 43.9% aqueous acetohydrazide solution (0.96 equivalent / hydrogen cyanide) prepared by the same method as in Example 7 was dropwise added within - 5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.43 equivalents relative to cyanogen chloride used in the reaction. After completion of the reaction, 32.3 g of water was added. The mixture was stirred for 15 minutes and was allowed to stand for 15 minutes, and the organic layer was taken and discarded.

Then, into a four-neck flask, 98.5 g of a 48% aqueous sodium hydroxide solution (1.60 equivalents / hydrogen cyanide) was put, and was stirred at room temperature, and the aqueous layer of the reaction liquid was dropwise added to the aqueous sodium hydroxide solution in the four-neck flask within 10 to 40⁰C to perform neutralization. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 46.1 g (yield 63.0%) of a white crystal of 2-amino-5-methyl-1,3,4-oxadiazole was obtained. The content of the product was 99.2% by mass, and the content of sodium chloride was 0.3% by mass.

### (Example 9)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of a 48% aqueous sodium hydroxide solution (0.80 equivalents / cyanogen chloride) and 64.9 g of a 77.0% acetohydrazide concentrated liquid (0.96 equivalents / hydrogen cyanide) prepared by the same method as in Example 1 were dropwise added simultaneously within -5 to 0⁰C over 1 over to adjust the byproduct hydrochloric acid in the liquid to 0.20 equivalents / cyanogen chloride after the dropwise addition. After completion of the dropwise addition, the reaction liquid was heated to 30⁰C, and was stirred at 30⁰C for 2 hours, whereby 2-amino-5-methyl-1,3,4-oxadiazole hydrochloride was synthesized. The amount of hydrochloric acid that did not form a salt on the completion of the reaction, that is, the byproduct hydrochloric acid in the step (A) was 0.20 equivalents relative to cyanogen chloride used in the reaction. After completion of the reaction, the reaction liquid was allowed to stand for 15 minutes.

Then, into a four-neck flask, 77.9 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 48.0 g (yield 68.9%) of a white crystal of 2-amino-5-methyl-1,3,4-oxadiazole was obtained. The content of the product was 99.4% by mass, and the content of sodium chloride was 0.2% by mass.

### (Example 10)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, 46.8 g of a 48% aqueous sodium hydroxide solution (0.80 equivalents / cyanogen chloride) was dropwise added within -5 to 0⁰C over 1 hour to adjust the byproduct hydrochloric acid in the liquid to 0.20 equivalents / cyanogen chloride after the dropwise addition. After completion of the dropwise addition, the reaction liquid was heated to 30⁰C, and was stirred at 30⁰C for 2 hours, whereby 2-amino-5-methyl-1,3,4-oxadiazole hydrochloride was synthesized. The amount of hydrochloric acid that did not form a salt on the completion of the reaction, that is, the byproduct hydrochloric acid in the step (A) was 0.20 equivalents relative to cyanogen chloride used in the reaction. After completion of the reaction, the reaction liquid was allowed to stand for 15 minutes.

Then, into a four-neck flask, 77.9 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 47.1 g (yield 67.6%) of a white crystal of 2-amino-5-methyl-1,3,4-oxadiazole was obtained. The content of the product was 99.5% by mass, and the content of sodium chloride was 0.3% by mass.

### (Example 11)

In a separable flask, 19.0 g of hydrogen cyanide (0.703 mol) was blown into 666.7 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of 48% sodium hydroxide (0.80 equivalents / cyanogen chloride) was dropwise added within -5 to 0⁰C over 1 hour to adjust the byproduct hydrochloric acid in the reaction liquid to 0.20 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.20 equivalents relative to cyanogen chloride used in the reaction. After completion of the reaction, the reaction liquid was allowed to stand for 15 minutes.

Then, into a four-neck flask, 77.9 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within 0 to 1⁰C for 1 hour. After stirring, a precipitated solid was taken by filtration, and was washed with cold water, whereby 38.0 g (yield 54.7%) of a white crystal of 2-amino-5-methyl-1,3,4-oxadiazole was obtained. The content of the product was 99.8% by mass, and the content of sodium chloride was 0.1% by mass.

### (Example 12)

In a separable flask, 19.0 g of hydrogen cyanide (0.703 mol) was blown into 130.3 g of water and 28.7 g of hexane, and the resultant was cooled to -5⁰C. After dropwise addition, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After confirming the completion of the reaction, 46.8 g of 48% sodium hydroxide (0.80 equivalents / cyanogen chloride) was dropwise added within -5 to 0⁰C over 1 hour to adjust the byproduct hydrochloric acid in the reaction liquid to 0.20 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.20 equivalents relative to cyanogen chloride used in the reaction. After completion of the reaction, the reaction liquid was allowed to stand for 15 minutes.

Then, into a four-neck flask, 77 g of a 48% aqueous sodium hydroxide solution (1.33 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was stirred within 32 to 35⁰C for 1 hour, and a precipitated solid was taken by filtration, and was washed with cold water, whereby 36.0 g (yield 51.7% / hydrogen cyanide) of a white crystal of 2-amino-5-methyl-1,3,4-oxadiazole was obtained. The content of the product was 99.3% by mass, and the content of NaCl was 0.2% by mass.

### (Comparative Example 1)

In a separable flask, 19.0 g of hydrogen cyanide (0.703 mol) was blown into 130.3 g of water and 28.7 g of hexane, and the resultant was cooled to -5⁰C. After cooling, 49.1 g of chlorine (0.985 equivalent / hydrogen cyanide) was blown within -5 to 0⁰C over 3 hours. After blowing, the liquid was stirred within -5 to 0⁰C for 40 minutes, and the reaction liquid was analyzed by gas chromatography, and then, it was found that 0.690 mol of cyanogen chloride was produced. After completion of the reaction, 26.4 g of a 48% aqueous sodium hydroxide solution (0.45 equivalents / cyanogen chloride) was dropwise added within -5 to 0⁰C over 1 hour to adjust the byproduct hydrochloric acid in the reaction liquid to 0.55 equivalents / cyanogen chloride. After neutralization of the byproduct hydrochloric acid, 64.9 g of a 77.0% acetohydrazide concentrated liquid (0.96 equivalents / hydrogen cyanide) prepared by the same method as in Example 1 was dropwise added within -5 to 5⁰C over 40 minutes. After dropwise addition, the reaction liquid was heated to 30⁰C, and was stirred at 30⁰C for 2 hours, whereby 2-amino-5-methyl-1,3,4-oxadiazole hydrochloride was synthesized. The byproduct hydrochloric acid on the completion of the reaction was 0.55 equivalents relative to cyanogen chloride used in the reaction. After completion of the reaction, the reaction liquid was allowed to stand for 15 minutes.

Then, into a four-neck flask, 98.4 g of a 48% aqueous sodium hydroxide solution (1.68 equivalents / hydrogen cyanide) was put, and was stirred at room temperature, and the aqueous layer of the reaction liquid in the separable flask was dropwise added to the aqueous sodium hydroxide solution in the four-neck flask within 10 to 40⁰C to perform neutralization. The remaining organic layer was separated and removed. The pH was 13 after the dropwise addition of the 48% aqueous sodium hydroxide solution. After neutralization, the resultant was cooled, and was stirred within -3 to 0⁰C for 1 hour. After stirring, a precipitated solid was taken by filtration, and was washed with cold water, whereby 34.5 g (yield 49.5%) of a white crystal of 2-amino-5-methyl-1,3,4-oxadiazole was obtained. The content of the product was 99.4% by mass, and the content of sodium chloride was 0.1% by mass.

## Claims

1. A method for producing a 2-amino-5-substituted-1,3,4-oxadiazole, the method comprising
(A) a step of preparing a cyanogen chloride solution from hydrogen cyanide and chlorine,
(B) a step of adjusting an amount of byproduct hydrochloric acid secondary produced together with cyanogen chloride in the step (A) to 0.5 equivalents or less relative to the cyanogen chloride produced,
(C) a step of subjecting a carboxylic acid hydrazide represented by the general formula (1) and the cyanogen chloride solution to a reaction to produce a solution containing a 2-amino-5-substituted-1,3,4-oxadiazole hydrochloride represented by the general formula (2): 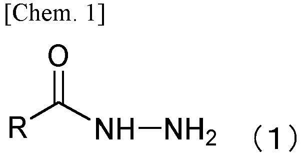 wherein R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group, 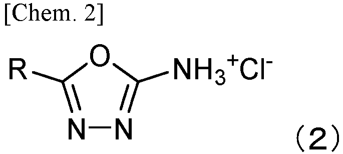 wherein R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group,
(D) a step of adding, to the solution obtained in the step (C), a base in an amount that is equal to or more than an equivalent obtained by adding up an amount of cyanogen chloride and an amount of the byproduct hydrochloric acid in the cyanogen chloride solution used in the step (C),
(E) a step of cooling a solution containing a hydrochloride of the base obtained in the step (D) and separating a precipitated crystal to thereby obtain a 2-amino-5-substituted-1,3,4-oxadiazole represented by the general (3): 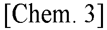 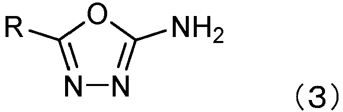 wherein R is a hydrogen atom, a C1 to C6 alkyl group, an aryl group, a 5- or 6-membered aromatic heterocyclic group, or an alkoxy group, which groups are optionally substituted with a halogen atom or a C1 to C4 alkyl group.

2. The method for producing a 2-amino-5-substituted-1,3,4-oxadiazole according to claim 1, wherein the cyanogen chloride solution in the step (A) is synthesized from chlorine and hydrogen cyanide of 1 equivalent or more relative to the chlorine.

3. The method for producing a 2-amino-5-substituted-1,3,4-oxadiazole according to claim 1, wherein the step (B) is a step in which a base is added to the cyanogen chloride solution obtained in the step (A) to prepare a cyanogen chloride solution in which the amount of the byproduct hydrochloric acid is made to be 0.5 equivalents or less relative to cyanogen chloride produced, and the cyanogen chloride solution prepared is used in the step (C).

4. The method for producing a 2-amino-5-substituted-1,3,4-oxadiazole according to claim 1, wherein the step (B) is a step in which the cyanogen chloride solution obtained in the step (A) is neutralized with a base under an organic solvent to prepare a cyanogen chloride solution in which the amount of the byproduct hydrochloric acid is made to be 0.5 equivalents or less relative to cyanogen chloride produced, and the cyanogen chloride solution prepared is used in the step (C).

5. The method for producing a 2-amino-5-substituted-1,3,4-oxadiazole according to claim 1, wherein the step (B) is a step in which the cyanogen chloride solution obtained in the step (A), a carboxylic acid hydrazide, and a base are mixed so that the amount of the byproduct hydrochloric acid present in the cyanogen chloride solution is made to be 0.5 equivalents or less relative to cyanogen chloride produced, and the reaction of the step (C) is performed with an obtained mixed liquid.

6. The method for producing a 2-amino-5-substituted-1,3,4-oxadiazole according to claim 1, wherein the step (B) is a step in which the cyanogen chloride solution obtained in the step (A) and a carboxylic acid hydrazide are mixed, and then, a base is mixed therewith so that the amount of the byproduct hydrochloric acid present in the cyanogen chloride solution is made to be 0.5 equivalents or less relative to cyanogen chloride produced, and the reaction of the step (C) is performed with an obtained mixed liquid.

7. The method for producing a 2-amino-5-substituted-1,3,4-oxadiazole according to claim 1, wherein the step (B) is a step in which cyanogen chloride is extracted from the cyanogen chloride solution obtained in the step (A) with an organic solvent to prepare a cyanogen chloride solution in which the amount of the byproduct hydrochloric acid is made to be 0.5 equivalents or less relative to cyanogen chloride produced, and the cyanogen chloride solution prepared is used in the step (C).

8. The method for producing a 2-amino-5-substituted-1,3,4-oxadiazole according to claim 1, wherein the solution in the step (E) has a concentration of the hydrochloride of the base of 10% by mass or more.

9. The method for producing a 2-amino-5-substituted-1,3,4-oxadiazole according to claim 1, wherein in the step (E), the crystal of a target product is separated at a temperature of 30⁰C or lower.
